# EUROPEAN PATENT SPECIFICATION

(11) **EP 1 476 032 B1**
(45) Date of publication and mention of the grant of the patent: **16.04.2008**
(21) Application number: 03702603.6
(22) Date of filing: 06.02.2003
(51) Int. Cl.: A23L 1/30, A61K 9/14, A61K 9/16

(54) **COENZYME Q10 FORMULATION**
COENZYM Q10 FORMULIERUNGEN
FORMULATIONS A BASE DE COENZYME Q10

(30) Priority: 14.02.2002 EP 02003433
(43) Date of publication of application: 17.11.2004
(73) Proprietor: DSM IP Assets B.V., 6411 TE Heerlen (NL)
(72) Inventor: CHEN, Chyi-Chen, CH-4102 Biningen (CH); LEUENBERGER, Bruno, CH-4123 Allschwil (CH); ULM, Johann, CH-4104 Oberwil (CH)
(74) Representative: Schwander, Kuno
(86) International application number: PCT/EP2003/001150
(87) International publication number: WO 2003/068008

(56) References cited:
- US-A- 5 478 569
- US-B1- 6 197 349
- DATABASE WPI Section Ch, Week 199503 Derwent Publications Ltd., London, GB; Class B05, AN 1995-021075 XP002238574 & SE 9 301 471 A (INST SOZIAL-MEDIZINISCHE FORSCHUNG), 30 October 1994 (1994-10-30)
- DATABASE WPI Section Ch, Week 198418 Derwent Publications Ltd., London, GB; Class B05, AN 1984-110774 XP002238575 & JP 59 051214 A (FREUND SANGYO KK), 24 March 1984 (1984-03-24)

## Description

The present invention relates to water-dispersible compositions comprising Coenzyme Q-10. Coenzyme Q-10 which by systematical nomenclature is 6-Decaprenyl-2,3-dimethoxy-5-methyl-1,4-benzoquinone, in view of its physiological activity, is used a supplement for human and animal nutrition. However, Coenzyme Q-10 is practically insoluble in water and therefore, has a poor bioavailability when administered orally. Efforts have been made to increase the solubility of Coenzyme Q-10, e.g., by means of forming solutions or emulsions using solubilizers e.g. surfactants such as polysorbates or lecithin see US 6,054,261 and US 4,483,873; polyethoxylated hydrogenated castor oil, see EP 522 433; or decaglyceryl stearate, see JP 2000212066; by providing stabilized fine aqueous dispersions of the solid compound, see US 6,197,349; or by forming micellar or liposomal aggregates of derivatives of the compound, see US 4,883,670.

SE-A 93-01471 discloses a basic preparation for incorporation in dentifrice for prophylactic and/or therapeutic treatment of gingivitis and paradontitis. The preparation contains an amount of the coenzyme Q10 and a protecting colloid-forming material. The preparation is in powder form with the coenzyme absorbed by dry particles of the colloid-forming material. The protective colloid-forming material is starch, particularly partially hydrolysed maize or potato starch, or it is gelatine. The coenzyme Q10 is the only component with gingiviral-protecting effects. The basic preparation also contains alpha-tocopherol in an amount effective as a free radial catchment agent. The dentifrice can be provided as a dental cream, containing a Bingham (RTM) fluid base and a grinding medium, and can also be in gel form, a tablet for a mouthwash, or even as a mouthwash itself.

JP 59-051214 discloses preparations of water-insoluble drugs with improved bioavailability. The preparation is prepared by spraying an O/W type emulsion of oil-soluble drugs, emulsifier and water-soluble macromolecules and drying it. Vitamin A, D, E or K, or ubiquinone-7, 9 or 10 are used as oil-soluble drugs. Polyglycerol fatty acid esters are used as emulsifier. 1-5 parts of emulsifier to I part of oil-soluble drugs (weight/weight) are used. Synthetic or semi-synthetic macromolecules, e.g. methylcellulose, polyvinyl alcohol and polyvinyl pyrrolidone, or natural macromolecules, e.g. gum arabic, gum tragacanth and gelatin, are used as water-soluble macromolecules. More than 3 parts of water-soluble macromolecules to 1 part of oil-soluble drugs (weight/weight) are used. The drugs exhibit high bioavailability. The drugs do not produce side effects such as hemolysis and irritation of the mucous membranes.

The present invention provides Coenzyme Q-10 formulations with improved bioavailability. In one aspect, the invention relates to novel powder compositions which comprise droplets of Coenzyme Q-10 which droplets are dispersed in a matrix of a modified polysaccharide containing a lipophilic moiety. The invention further relates to novel aqueous emulsion compositions comprising droplets of coenzyme Q-10 which droplets are dispersed in a modified polysaccharide matrix. Still further, the invention relates to food, beverages, feeds, cosmetics and pharmaceutical products comprising such Coenzyme Q-10 formulation.

In another aspect, the invention relates to a novel process for the preparation of novel powder compositions which comprise droplets of Coenzyme Q-10 which droplets are dispersed in a matrix of a modified polysaccharide.

The term "modified polysaccharide" refers to polysaccharides which contain a lipophilic moiety, e.g., a hydrocarbon moiety having a chain length of preferably 5 to 18 carbon atoms in the straight chain. Preferably the modified polysaccharide should be at least acceptable for animal consumption. For human consumption, preferred modified polysaccharides should be GRAS (generally recognized as safe) or an approved material for food consumption as determined by the various regulatory agencies world wide. A preferred modified polysaccharide is modified starch. Starches are hydrophilic and, therefore, do not have emulsifying capacities. However, modified starches are made from starches substituted by known chemical methods with hydrophobic moieties. For example starch may be treated with cyclic dicarboxylic acid anhydrides such as succinic and glutaric anhydrides, substituted with an alkyl or alkenyl group. A particularly preferred modified starch of this invention has a structure as depicted below 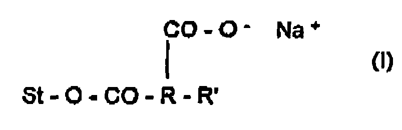 wherein St is a starch, R is an alkylene radical and R' is a hydrophobic group. Preferably the alkylene radical is lower alkylene, such as dimethylene or trimethylene. R' may be an alkyl or alkenyl group, preferably containing 5 to 18 carbon atoms. A preferred modified starch of formula I is starch sodium octenyl succinate. It is available commercially from, among other sources, National Starch and Chemical Company, Bridgewater, N.J. as Capsul^{®}. Making this compound, and the compounds of Formula 1 in general, is known in the art (see "Modified Starches: Properties and Uses, ed. O.B. Wurzburg, CRC Press, Inc., Boca Raton, Florida (1991))

The composition of this invention may comprise up to about 60 % by weight of Coenzyme Q-10 in the matrix on a dry weight basis. Preferably, the percentage of Coenzyme Q-10 in the matrix is from about 10% to about 40 %, most preferably about 25% by weight.

The composition may also contain a small amount of residual water. The amount of residual water depends on the drying technology used, which will be evident to a skilled practitioner. The amount of residual water also depends on the proportion of the matrix material in the composition and may be up to about 10% of a composition having a high matrix proportion, and typically is about 5 % by weight. Alternatively, other ingredients standard to a powder composition may be added, for example sucrose, maltodextrin or antioxidants alone or in combination, and the amounts of Coenzyme Q-10 and polysaccharide or gelatin adjusted accordingly.

The novel powder compositions according to the present invention can be produced by a process which comprises:
a) providing an aqueous solution of a modified polysaccharide containing a lipophilic moiety;
b) adding coenzyme Q-10 to the solution of step a);
c) emulsifying the product of step b) to obtain an emulsion of the desired size of the droplets; and
d) drying the emulsion of step c) to obtain a powder composition.

In one embodiment of the present invention, coenzyme Q-10 is heated to obtain a melt and is then added in molten form to a slightly warmed (e.g., at about 40 to 50°C) solution of the matrix component, i.e. the modified polysaccharide. Suitably, an antioxidant such as dl-tocopherol is added to the Coenzyme Q-10 melt. If desired, further components such as sucrose or maltodextrin maybe added to the matrix solution. The mixture is then emulsified using conventional equipment until the desired size of the emulsion particles is obtained.

In a particular embodiment of the process of this invention, the powder composition is produced by
aa) providing an aqueous solution of a modified polysaccharide containing a lipophilic moiety;
bb) adding Coenzym Q-10 in molten form to an aqueous solution of such a modified polysaccharide with mixing to form a crude emulsion, preferably a pre-emulsion having a solids content of from about 30% to about 50%, more preferably of about 45%; wherein the emulsion droplets have an average diameter of 2000 nm or smaller;
cc) emulsifying the crude emulsion further in a high-pressure homogenizer at a temperature of about 5°C to about 75°C and at a pressure of about 600 bar to about 4000 bar; and
dd) spray-drying the emulsion.

Step a) and aa), respectively, can be done at any reasonable temperature to ensure a rapid dissolution of the modified polysaccharide in water and to fully utilize its functionality.

To ensure complete dissolution of modified polysaccharide within a reasonable amount of time, heating to about 40°or 80°C is preferable, after which the resulting solution may be conveniently cooled to about 40°C. Coenzym Q-10 is then added in molten form and the mixture is homogenized to a crude emulsion (for example by using a colloid mill or any other conventional mixing means) until the droplet size is preferably not greater than 2000 nm. Droplet size may be measured by any conventional particle size analyzer. A preferred measuring means is laser light scattering technique. The Malvern ZetaSizer (Malvern Instruments, Southborough, MA) is an example of a laser light scattering measuring device.

The crude emulsion is then further emulsified using high pressure homogenizing equipment and vessels for this purpose. The device selected should provide a sufficiently high pressure. The high pressure homogenizing device, such as model DeBEE 2000 from BEE International (Migdal Ha'emek, Israel), is suitable for this purpose. It is also possible to use a water jet (such as those produced by Jet Edge Inc., Minneapolis, MN). The crude emulsion may be transferred from the holding vessel to the emulsifying device through a suitable sieve in order to prevent from clogging of the high pressure homogenizer. The temperature at which the homogenization (by which is meant further emulsification) takes place is best kept above room temperature e.g., between about 30° up to about 75° with a cooling system such as an ice water bath to control the temperature at high pressures. The pressure pump of the emulsifying device should be set at a suitable pressure (600 to 4000 bar depending on the desired droplet size).

Homogenization continues for a sufficient number of passes to obtain an emulsion of the desired droplet size. In general, the stabler the process pressure, the fewer number of passes should be required to achieve the same droplet size. The emulsion is then dried to obtain the powder of this invention. Drying may be accomplished by any standard method, for example spray-drying in a suitable spray dryer, such as Model Mobile Minor from Niro/AS (Soeborg, Denmark).

In order to attain the desired droplet size, the emulsion step cc) may be repeated through one or more passes as necessary to obtain the desired droplet size, i.e. the crude emulsion is passed into the homogenization vessel, emulsified, passed out of the homogenization vessel, and passed through the homogenization vessel again until the desired droplet size is attained. Usually up to twenty passes will be required depending on the emulsification pressure used. These passes are usually all performed at the same pressure and the same system parameters, but different pressures may be used for different passes (other system parameters could also be varied for different passes). The period of time for one pass is not critical. The amount of time per pass will depend on system parameters including emulsion viscosity, batch size, flow rate and pressure. These parameters will depend on the precise processing format selected, and may be varied by the skilled person to obtain the desired results. Emulsification passes should continue until testing shows that the desired droplet size is achieved as determined by particle size analysis. Suitably, the average droplet size of the Coenzyme Q-10 emulsion of this invention is not greater than about 500 nm, preferably less than about 200 nm and most preferably 130 nm or less. To obtain a droplet diameter of 130 nm or less it is important that the homogenization step be performed at an ultra-high pressure as described above to effectively reduce the droplet size of the emulsion to a desirable size. The pressure used in the homogenizer is preferably higher than 1000 bar and most preferably higher than 1500 bar. The homogenization temperature as measured at the exit of the homogenizer is preferably below 70° C. The emulsion is then converted to a powder, by a known technology such as freeze-drying, fluid-bed drying, beadlet formation, but preferably by spray-drying, to obtain a powder composition which comprises droplets of Coenzym Q-10 having a diameter of not greater than about 500 nm, preferably less than about 200 nm and most preferably 130 nm or less and which are dispersed in the matrix.

The Coenzyme Q-10 compositions of this invention can be used to fortify food and beverages. They can also be used in pharmaceutical compositions designed to provide a supply for Coenzyme Q-10. Beverages fortified with Coenzyme Q-10 can be obtained by adding to a beverage a powder composition of this invention. Adding a powder composition of this invention to a liquid requires no special procedure or extensive mixing. The powder may simply be added to the liquid and mixed by shaking or stirring until the powder particles are no longer visible to the naked eye.

The powder compositions of this invention may also be added to cosmetics if it is desired to blend Coenzyme Q-10 into a cosmetic. If the cosmetic is optically clear, preferred compositions of this invention may be used to avoid increasing the turbidity of the cosmetic. Cosmetics include any materials designed for application to the skin, hair, or nails, for example skin care products such as balms, lotions, or sticks, various ointments, make-up compositions for use on the face, eyes, or lips, shampoos and conditioners, nail polishes, and the like. The cosmetic may contain other active ingredients. Pharmaceutical compositions intended for topical application in the form of ointments, lotions, and the like are also contemplated. Cosmetic formulations will be well known to the skilled person. The powder composition of this invention is added at an appropriate time in the production process such as to be thoroughly blended into the cosmetic.

The following examples serve to demonstrate the present invention, but are not intended to limit it in any way.

### Example 1

58 g of fish gelatin (bloom number 0) and 58 g of sucrose were placed in a 500-ml vessel and 60 ml of de-ionized water was then added. The mixture was brought into solution while stirring with a mincer disc. at 1000 rpm at 40°C, which gave the matrix 40 g of coenzyme Q-10, mixed with 2 g of dl-α-tocopherol, was melted at 55°C. Thereupon, this mixture were emulsified in the matrix and stirred for 15 minutes. During the emulsification and stirring the mincer disc was rotated at 4800 rpm. After this time the internal phase of the emulsion had an average particle size of about 260 nm. The emulsion was then diluted with 70 ml of de-ionized water and heated to 60°C. Subsequently 1300 g of corn starch were placed in a laboratory spray tank and cooled to at least 0°C. The emulsion was sprayed into the spray tank using a rotating spray nozzle. The thus-obtained particles plus the corn starch were sieved (sieve fraction 160 to 630 µm) to remove the excess corn starch and the particles were then dried at room temperature using a stream of air to yield 237 g dry particles, which contained 16.3% coenzyme Q-10.

### Example 2

In an experiment analogous to Example 1, the fish gelatin was replaced with a starch sodium octenyl succinate (National Starch and Chemical Company, Bridgewater, N.J.). The particle size of the inner phase was about 160 nm. The yield was 288 g and the coenzyme Q-10 content was 13.4 %.

### Examples 3

267.4 g of starch sodium octenyl succinate (National Starch and Chemical Company, Bridgewater, N.J.) is dissolved in 287.8 g of deionized water at 80 °C. After the starch solution is cooled to about 40 °C, a solution of 50 g of coenzyme Q-10 and 2.5 g of dl-α-tocopherol, which is prepared at 55 °C, is gradually added to the starch sodium octenyl succinate solution and homogenized with a lab homogenizer until the droplet size of the emulsion is not greater than 2000 nm. The crude emulsion is then homogenized with a high pressure homogenizer at 2000 bar. The emulsion is recycled through the homogenization process until the droplets of the emulsion reaches 130 nm or below.

The emulsion may be spray-dried (Niro A/S, Soeorg, Denmark, Model Mobile Minor) to yield a powder containing about 15% of Coenzyme Q-10.

## Claims

1. A powder composition which comprises droplets of Coenzyme Q-10 which droplets are dispersed in a matrix of a modified polysaccharide containing a lipophilic moiety.

2. A powder composition of claim 1 wherein the modified polysaccharide is a modified starch.

3. A powder composition of claim 2 wherein the modified starch has the structure St - O - CO - R (R') - CO - O⁻ Na⁺ (I), wherein St is a starch, R is an alkylene radical and R' is a hydrophobic group.

4. A powder composition of claim 2 or claim 3 wherein the modified starch is starch sodium octenyl succinate.

5. A powder composition of any one of claims 1 to 4 wherein the matrix further comprises a mono- or disaccharide, or a polysaccharide.

6. A powder composition of claim 5 wherein the matrix further comprises sucrose.

7. A powder composition of claim 5 wherein the matrix further comprises maltodextrin.

8. A powder composition of any one of claims I to 7 wherein the amount of coenzyme Q-10 is from about 1% to about 60% by weight (based on dry weight).

9. A powder composition of any one of claims 1 to 7 wherein the amount of coenzyme Q-10 is from about 10% to about 40% by weight (based on dry weight).

10. An aqueous emulsion composition which comprises droplets of coenzyme Q-10 which droplets are dispersed in an aqueous solution of a modified polysaccharide containing a lipophilic moiety.

11. An emulsion composition of claim 10 wherein the average droplet diameter is about 500 nm or less.

12. An emulsion composition of claim 10 wherein the average droplet diameter is about 200 nm or less.

13. An emulsion composition of claim 10 wherein the average droplet diameter is about 130 nm or less.

14. A beverage or food comprising a powder composition of any one of claims 1 to 9 or an emulsion of any one of claims 10 to 13.

15. A process for producing a powder composition which comprises droplets of coenzyme Q-10 and which are dispersed in a matrix of a modified polysaccharide containing a lipophilic moiety, which comprises
a) providing an aqueous solution of such a modified polysaccharide;
b) adding coenzyme Q-10 to the solution of step a);
c) emulsifying the product of step b) to obtain an emulsion of the desired size of the droplets; and
d) drying the emulsion of step c) to obtain a powder composition.

16. The process of claims 14 which comprises
aa) providing an aqueous solution of a modified polysaccharide containing a lipophilic moiety;
bb) adding Coenzym Q-10 in molten form to an aqueous solution of such a modified polysaccharide with mixing to form a crude emulsion, preferably a pre-emulsion having a solids content of from about 30% to about 50%, more preferably of about 45%; wherein the emulsion droplets have an average diameter of 2000 nm or smaller;
cc) emulsifying the crude emulsion further in a high-pressure homogenizer at a temperature of about 5°C to about 75°C and at a pressure of about 600 bar to about 4000 bar; and
dd) spray-drying the emulsion.

## Patentansprüche

1. Pulverzusammensetzung, umfassend Tröpfchen von Coenzym Q-10, wobei die Tröpfchen in einer Matrix eines modifizierten Polysaccharids dispergiert sind, das eine lipophile Einheit enthält.

2. Pulverzusammensetzung nach Anspruch 1, wobei das modifizierte Polysaccharid eine modifizierte Stärke ist.

3. Pulverzusammensetzung nach Anspruch 2, wobei die modifizierte Stärke die Struktur St-O-CO-R(R')-CO-O⁻Na⁺ (I) hat, wobei St eine Stärke ist, R ein Alkylenradikal ist, und R' eine hydrophobe Gruppe ist.

4. Pulverzusammensetzung nach Anspruch 2 oder Anspruch 3, wobei die modifizierte Stärke Stärke-Natriumoctenylsuccinat ist.

5. Pulverzusammensetzung nach einem der Ansprüche 1 bis 4, wobei die Matrix zudem ein Mono- oder Disaccharid oder ein Polysaccharid umfasst.

6. Pulverzusammensetzung nach Anspruch 5, wobei die Matrix zudem Saccharose umfasst.

7. Pulverzusammensetzung nach Anspruch 5, wobei die Matrix zudem Maltodextrin umfasst.

8. Pulverzusammensetzung nach einem der Ansprüche 1 bis 7, wobei die Menge an Coenzym Q-10 von etwa 1 Gew.-% bis etwa 60 Gew.-% (bezogen auf das Trockengewicht) reicht.

9. Pulverzusammensetzung nach einem der Ansprüche 1 bis 7, wobei die Menge an Coenzym Q-10 von etwa 10 Gew.-% bis etwa 40 Gew.-% (bezogen auf das Trockengewicht) reicht.

10. Wässrige Emulsionszusammensetzung, umfassend Tröpfchen von Coenzym Q-10, wobei die Tröpfchen in einer wässrigen Lösung eines modifizierten Polysaccharids dispergiert sind, das eine lipophile Einheit enthält.

11. Emulsionszusammensetzung nach Anspruch 10, wobei der mittlere Tröpfchendurchmesser etwa 500 nm oder kleiner ist.

12. Emulsionszusammensetzung nach Anspruch 10, wobei der mittlere Tröpfchendurchmesser etwa 200 nm oder kleiner ist.

13. Emulsionszusammensetzung nach Anspruch 10, wobei der mittlere Tröpfchendurchmesser etwa 130 nm oder kleiner ist.

14. Getränk oder Nahrungsmittel, umfassend eine Pulverzusammensetzung nach einem der Ansprüche 1 bis 9 oder eine Emulsion nach einem der Ansprüche 10 bis 13.

15. Verfahren zur Herstellung einer Pulverzusammensetzung, umfassend Tröpfchen von Coenzym Q-10, die in einer Matrix eines modifizierten Polysaccharids dispergiert sind, das eine lipophile Einheit enthält, umfassend:
a) Bereitstellen einer wässrigen Lösung eines solchen modifizierten Polysaccharids;
b) Zugeben von Coenzym Q-10 zur Lösung von Schritt a);
c) Emulgieren des Produktes von Schritt b) zur Gewinnung einer Emulsion mit der gewünschten Tröpfchengröße; und
d) Trocknen der Emulsion von Schritt c) zur Gewinnung einer Pulverzusammensetzung.

16. Verfahren nach Anspruch 14, umfassend:
aa) Bereitstellen einer wässrigen Lösung eines modifizierten Polysaccharids, das eine lipophile Einheit enthält;
bb) Zugeben von Coenzym Q-10 in geschmolzener Form zu einer wässrigen Lösung eines solchen modifizierten Polysaccharids unter Mischen, so dass man eine rohe Emulsion erhält, vorzugsweise eine Voremulsion mit einem Feststoffgehalt von etwa 30% bis etwa 50%, stärker bevorzugt etwa 45%; wobei die Emulsionströpfchen einen mittleren Durchmesser von 2000 nm oder weniger aufweisen;
cc) weiteres Emulgieren der rohen Emulsion in einem Hochdruck-Homogenisator bei einer Temperatur von etwa 5°C bis etwa 75°C und bei einem Druck von etwa 600 bar bis etwa 4000 bar; und
dd) Sprühtrocknen der Emulsion.

## Revendications

1. Composition de poudre qui comprend des gouttelettes de la coenzyme Q-10, lesquelles gouttelettes sont dispersées dans une matrice d'un polysaccharide modifié qui comprend un groupement lipophile.

2. Composition de poudre selon la revendication 1, dans laquelle le polysaccharide modifié est un amidon modifié.

3. Composition de poudre selon la revendication 2, dans laquelle l'amidon modifié présente la structure St-O-CO-R(R')-CO-O⁻Na⁺ (I), dans laquelle St représente un amidon, R représente un radical alkylène et R' représente un groupe hydrophobe.

4. Composition de poudre selon la revendication 2 ou la revendication 3, dans laquelle l'amidon modifié est de l'octényle succinate d'amidon sonique.

5. Composition de poudre selon l'une quelconque des revendications 1 à 4, dans laquelle la matrice comprend en outre un monosaccharide, un disaccharide ou un polysaccharide.

6. Composition de poudre selon la revendication 5, dans laquelle la matrice comprend en outre du saccharose.

7. Composition de poudre selon la revendication 5, dans laquelle la matrice comprend en outre de la maltodextrine.

8. Composition de poudre selon l'une quelconque des revendications 1 à 7, dans laquelle la quantité de coenzyme Q-10 varie d'environ 1 % à environ 60 % en poids (basé sur le poids sec).

9. Composition de poudre selon l'une quelconque des revendications 1 à 7, dans laquelle la quantité de coenzyme Q-10 varie d'environ 10 % à environ 40 % en poids (basé sur le poids sec).

10. Composition d'émulsion aqueuse qui comprend des gouttelettes de la coenzyme Q-10, lesquelles gouttelettes sont dispersées dans une solution aqueuse d'un polysaccharide modifié qui comprend un groupement lipophile.

11. Composition d'émulsion selon la revendication 10, dans laquelle le diamètre moyen des gouttelettes est d'environ 500 nm ou moins.

12. Composition d'émulsion selon la revendication 10, dans laquelle le diamètre moyen des gouttelettes est d'environ 200 nm ou moins.

13. Composition d'émulsion selon la revendication 10, dans laquelle le diamètre moyen des gouttelettes est d'environ 130 nm ou moins.

14. une boisson ou un aliment qui comprend une composition de poudre selon l'une quelconque des revendications 1 à 9 ou une émulsion selon l'une quelconque des revendications 10 à 13.

15. Procédé de fabrication d'une composition de poudre qui comprend des gouttelettes de la coenzyme Q-10, lesquelles sont dispersées dans une matrice d'un polysaccharide modifié qui comprend un groupement lipophile, lequel comprend :
a) la fourniture d'une solution aqueuse d'un tel polysaccharide modifié ;
b) l'ajout de la coenzyme Q-10 à la solution de l'étape a) ;
c) l'émulsification du produit de l'étape b) pour obtenir une émulsion avec la dimension souhaitée de gouttelettes ; et
d) le séchage de l'émulsion de l'étape c) pour obtenir une composition de poudre.

16. Procédé selon la revendication 14, lequel comprend :
aa) la fourniture d'une solution aqueuse d'un polysaccharide modifié qui comprend un groupement lipophile ;
bb) l'ajout de la coenzyme Q-10 sous une forme fondue à une solution aqueuse d'un tel polysaccharide modifié avec mélange pour former une émulsion brute, de préférence une préémulsion ayant une teneur en solides d'environ 30 % à environ 50 %, plus préférablement d'environ 45 % ; dans lequel les gouttelettes d'émulsion ont un diamètre moyen de 2000 nm ou moins ;
cc) une autre émulsification de l'émulsion brute dans un homogénéisateur à haute pression à une température d'environ 5 °C à environ 75 °C et à une pression d'environ 600 bars à environ 4000 bars ; et
dd) le séchage par atomisation de l'émulsion.
